# EUROPÄISCHE PATENTSCHRIFT

(11) **EP 1 465 857 B1**
(45) Veröffentlichungstag und Bekanntmachung des Hinweises auf die Patenterteilung: **07.04.2010**
(21) Anmeldenummer: 03702381.9
(22) Anmeldetag: 03.01.2003
(51) Int. Cl.: C07C 51/50, C07C 57/075

(54) **VERFAHREN ZUR HERSTELLUNG VON ACRYSÄURE UND POLYMERISIERBARE VERBINDUNGEN**
METHOD FOR PRODUCING ACRYLIC ACID AND POLYMERISABLE COMPOUNDS
PROCEDE DE PRODUCTION D'ACIDE ACRYLIQUE ET D'AUTRES COMPOSES POLYMERISABLES

(30) Priorität: 09.01.2002 DE 10200583; 17.04.2002 DE 10217121
(43) Veröffentlichungstag der Anmeldung: 13.10.2004
(73) Patentinhaber: BASF SE, 67056 Ludwigshafen (DE)
(72) Erfinder: HAMMON, Ulrich, 68163 Mannheim (DE); SCHRÖDER, Jürgen, 67071 Ludwigshafen (DE)
(86) Internationale Anmeldenummer: PCT/EP2003/000010
(87) Internationale Veröffentlichungsnummer: WO 2003/057657

(56) Entgegenhaltungen:
- WO-A-99/14182
- DE-A- 10 064 641
- US-A- 5 322 960
- US-B1- 6 210 536
- DATABASE WPI Section Ch, Week 199621 Derwent Publications Ltd., London, GB; Class A41, AN 1996-205483 XP002239464 & JP 08 073405 A (NIPPON SHOKUBAI CO LTD), 19. März 1996 (1996-03-19)

## Beschreibung

Die vorliegende Erfindung betrifft ein Verfahren zur fraktionierenden Kondensation eines Acrylsäure enthaltenden heißen Gasgemischs.

Aus der DE-A 197 40 253 und der DE-A 102 35 847 ist bekannt, daß ein Reaktionsgemisch aus der katalytischen Gasphasenoxidation zur Herstellung von Acrylsäure fraktionierend kondensiert werden kann, indem man es von unten in eine Kolonne mit trennwirksamen Einbauten führt und die kondensierbaren Komponenten durch Kühlung auskondensiert.

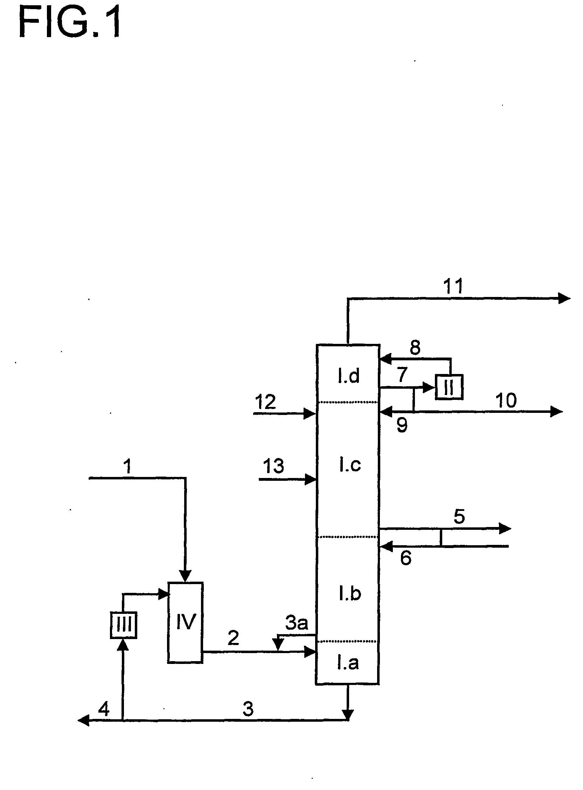

In letzterem Verfahren (Fig. 1) wird die Kolonne bevorzugt auf folgende Weise stabilisiert :
Der obere Kolonnenbereich wird mit einer wasserlöslichen phenolischen Verbindung, vorzugsweise Hydrochinonmonomethylether, stabilisiert. Die Zugabe des Stabilisators erfolgt als Lösung in Sauerwasser in den Rücklaufstrom bzw. in den Quenchkreislauf.

Der übrige Kolonnenbereich wird mit einer 0,1 - 1 Gew.%igen Lösung von Phenothiazin in Acrylsäure stabilisiert, wobei die Zugabe in dem Kolonnenbereich erfolgt, in dem die Acrylsäurekonzentration 5 - 15 %, bzw. die Wasserkonzentration 80 - 95 % beträgt. Die zugabemenge wird so bemessen, daß der Phenothiazingehalt in der Acrylsäurefraktion (Mittelsiederfraktion, Strom 7) 10 - 1000 ppm, vorzugsweise 50 - 500 ppm beträgt.

Nachteilig an dem dort dargestellten Verfahren ist, daß die wasserlöslichen phenolischen Verbindungen, insbesondere Hydrochinonmonomethylether, im als Lösungsmittel verwendeten Sauerwasser, das als Leichtsiederfraktion anfällt, nur begrenzt löslich sind. Dadurch werden große Mengen an Stabilisatorlösung benötigt.

Weiterhin nachteilig ist, daß der effektive, aber wenig wasserlösliche Stabilisator Phenothiazin wegen der dortigen hohen Wasserkonzentration nicht in den oberen Kolonnenbereich dosiert werden kann. Als gängige Lösungsmittel für Phenothiazin kommen nur das Sauerwasser oder Acrylsäure in Frage. Andere Lösungsmittel bringen fremde Stoffe in das System und sind daher unerwünscht. Phenothiazin ist im Sauerwasser praktisch unlöslich. Mit Acrylsäure als Lösungsmittel ginge die dafür verwendete Acrylsäure über das Sauerwasser verloren.

US 6,210,536 offenbart die Verwendung von N,N-Dinitrosophenyldiaminen als Polymerisationsinhibitoren für Acrylsäure und deren Estern. Auch der Einsatz von Phenothiazin gemeinsam mit Hydrochinonmonomethylether wird offenbart, jedoch nur als Feststoff oder gelöst in Toluol oder Acrylsäure.

Aus der US 5,322,960 ist ein System aus drei Polymerisationsinhibitoren für (Meth)acrylsäure und deren Ester bekannt. Dieses System setzt sich aus einer N-Oxcylverbindung, einer Phenolverbindung und Phenothiazin zusammen und wird entweder als Feststoff, in wässriger Lösung oder in einem anderen Lösungsmittel eingesetzt.

Der vorliegenden Erfindung lag nun die Aufgabe zugrunde, ein Verfahren zu entwickeln, in dem mit Phenothiazin bei der Herstellung von Acrylsäure auch in Bereichen stabilisiert werden kann, in denen eine hohe Wasserkonzentration vorliegt.

Die Aufgabe wird erfindungsgemäß gelöst durch ein Verfahren zur fraktionierenden Kondensation eines heißen Gasgemischs gefunden, das neben Acrylsäure wenigstens eine weitere kondensierbare Komponente enthält, in einer Kolonne in Anwesenheit mindestens eines Stabilisators, in dem der mindestens eine Stabilisator zumindest teilweise als Schmelze dosiert wird.

Das erfindungsgemäße Verfahren wird im allgemeinen wie folgt durchgeführt:
Als heiße Gasgemische sind solche Gasgemische geeignet wie das Reaktionsgasgemisch, das bei der katalytischen Gasphasenoxidation von C₃-Alkanen, -Alkenen, -Alkanolen und/oder -Alkanalen und/oder Vorstufen davon zu Acrylsäure nach bekannten Verfahren entsteht. Besonders vorteilhaft wird Propen, Propan und Acrolein zur Herstellung von Acrylsäure eingesetzt. Als Ausgangsverbindungen für Acrylsäure sind aber auch solche verwendbar, aus denen sich die eigentliche C₃- Ausgangsverbindung erst während der Gasphasenoxidation intermediär bildet. Acrylsäure kann direkt aus Propan hergestellt werden. Bei Einsatz von Propan als Ausgangsstoff kann dieses nach bekannten Verfahren durch katalytische Oxidehydrierung, homogene Oxidehydrierung oder katalytische Dehydrierung zu einem Propen-/Propan-Gemisch umgesetzt werden. Geeignete Propen-/Propan-Gemische sind auch Raffineriepropen (ca. 70 % Propen und 30 % Propan) oder Crackerpropen (ca. 9.5 % Propen und 5 % Propan). Bei Einsatz eines Propen-/Propan-Gemisches zur Herstellung von Acrylsäure wirkt Propan als Verdünnungsgas und/oder Reaktand. Bei der Herstellung der Acrylsäure werden in der Regel die Ausgangsgase mit unter den gewählten Reaktionsbedingungen inerten Gasen wie Stickstoff (N₂), CO₂, gesättigten C₁-C₆-Kohlenwasserstoffen und/oder Wasserdampf verdünnt und im Gemisch mit Sauerstoff (O₂) oder einem sauerstoffhaltigen Gas bei erhöhten Temperaturen (üblicherweise 200 bis 450 °C) sowie gegebenenfalls erhöhtem Druck über übergangsmetallische (z.B. Mo, V, W und/oder Fe enthaltende) Mischoxidkatalysatoren geleitet und oxidativ in die Acrylsäure umgewandelt. Diese Umsetzungen werden beispielsweise einstufig oder mehrstufig durchgeführt.

Das entstehende Reaktionsgasgemisch enthält neben der gewünschten Säure Nebenkomponenten wie nicht umgesetztes Acrolein und/oder Propen, Wasserdampf, Kohlenmonoxid, Kohlendioxid, Stickstoff, Sauerstoff, Essigsäure, Propionsäure, Formaldehyd, weitere Aldehyde und Maleinsäure beziehungsweise Maleinsäureanhydrid. Üblicherweise enthält das Reaktionsgasgemisch, jeweils bezogen auf das gesamte Reaktionsgasgemisch, 1 bis 30 Gew.-% Acrylsäure, 0,01 bis 1 Gew.-% Propen und 0,05 bis 1 Gew.-% Acrolein, 0,05 bis 10 Gew.-% Sauerstoff, 0,01 bis 3 Gew.-% Essigsäure, 0,01 bis 2 Gew.-% Propionsäure, 0,05 bis 1 Gew.-% Formaldehyd, 0,05 bis 2 Gew.-% sonstige Aldehyde, 0,01 bis 0,5 Gew.-% Maleinsäure und Maleinsäureanhydrid und als Restmenge inerte Verdünnungsgase. Als inerte Verdünnungsgase sind insbesondere gesättigte C₁ - C₆-Kohlenwasserstoffe, wie Methan und/oder Propan, daneben Wasserdampf, Kohlenoxide und Stickstoff enthalten. Somit enthält ein solches Gasgemisch neben der Zielkomponente Acrylsäure, die überwiegend als Mittelsiederfraktion kondensiert, weitere Verbindungen im Schwersieder- und Leichtsiederbereich sowie nicht kondensierbare Anteile. Die Mittelsiederfraktion umfaßt im wesentlichen die Komponenten, die bei Normaldruck einen Siedepunkt im Temperaturintervall im Fall von Acrylsäure beispielsweise von 120 bis 180 °C, insbesondere im Bereich von +/- 10 °C um den des Wertproduktes, d.h. bei Acrylsäure etwa 131 bis 151 °C aufweisen.

Die Abkühlung des Reaktionsgasgemisches kann indirekt, beispielsweise durch Wärmetauscher, die dem Fachmann an sich bekannt sind und keiner Beschränkung unterliegen, oder direkt, beispielsweise durch einen Quench, erfolgen, vorzugsweise durch direkte Kühlung.

Diese kann sowohl im Sumpfbereich der Kolonne als auch getrennt von der Kolonne in einem separaten Apparat IV erfolgen, wie es in den Figuren 1 bis 5 gezeigt ist. In diesem Fall wird das heiße, zu kondensierende Gasgemisch mit einer Temperatur zwischen 200 und 400 °C aus Leitung 1 in einem Quench IV üblicherweise auf eine Temperatur zwischen 100 und 180 °C abgekühlt und über Leitung 2 dem Sumpfbereich I.a der Kolonne zugeführt. Über Leitung 3 wird das Kühlmedium (nicht verdampfte Schwersiederfraktion aus I.a) zur Kühlung des heißen Gasgemisches in den Quench IV zurückgeführt.

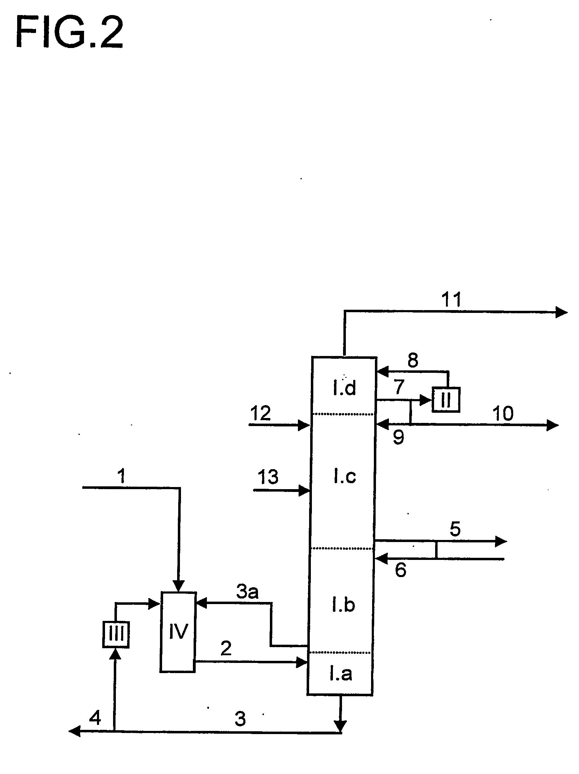

In einer besonderen Ausführungsform können die Schwersiederfraktionen aus I.a und I.b getrennt in den Quench zurückgeführt werden (Fig. 2, Strom 3 und Strom 3a). Besonders bevorzugt wird dabei die Schwersiederfraktion aus I.b direkt, ohne Wärmetauscher, dem Apparat IV zugeführt (Fig. 2, Strom 3a).

Ein Teil des Kühlmediums aus dem Kreislauf III/IV, üblicherweise 0,5 bis 5 Gew.-% bezogen auf 100 Gew.-% Kondensat im Seitenabzug, kann aus dem Prozeß ausgeschleust werden (Strom 4). Dieser Strom hat im Fall der Herstellung von Acrylsäure in der Regel folgende Zusammensetzung:

| | |
|---|---|
| 10 - 40 Gew.% | Acrylsäure |
| 10 - 40 Gew.% | Diacrylsäure |
| 5 - 15 Gew.% | Maleinsäure / Maleinsäureanhydrid |
| 1 - 3 Gew.% | Benzoesäure |
| 2 - 6 Gew.% | Phthalsäure / Phthalsäureanhydrid |
| Rest: | Stabilisatoren, polymere Acrylsäure, höhermolekulare Michael-Additionsprodukte der Acrylsäure (Tri- , Tetraacrylsäure etc.) |

Ausschleusung kann dabei Entsorgung, z.B. Verbrennung, bedeuten, oder der ausgeschleuste Strom kann z.B. einer thermischen und/oder katalytischen Behandlung, z.B. zum Zwecke der Spaltung von Hochsiedern, unterworfen werden, deren abgetrennten Spaltprodukte wieder an beliebiger Stelle in den erfindungsgemäßen Prozeß eingespeist werden können.

Als Quenchvorrichtung IV können alle im Stand der Technik für diesen Zweck bekannten Vorrichtungen (z.B. Sprühwasser, Venturiwäscher, Blasensäulen oder sonstige Apparate mit berieselten Oberflächen) eingesetzt werden, wobei vorzugsweise Venturi-Wäscher oder Sprühkühler verwendet werden.

Zur indirekten Kühlung oder Erwärmung der Quenchflüssigkeit eignen sich alle gängigen Wärmeüberträger oder Wärmetauscher. Als bevorzugt seien Rohrbündelwärmetauscher, Plattenwärmetauscher und Luftkühler genannt. Die Temperatur der Quenchflüssigkeit beträgt nach Verlassen des Wärmetauschers III normalerweise 70 bis 200°C, häufig 100 bis 150°C. Geeignete Kühlmedien sind Luft bei einem entsprechenden Luftkühler und Kühlflüssigkeiten, insbesondere Wasser, bei den anderen Kühlvorrichtungen.

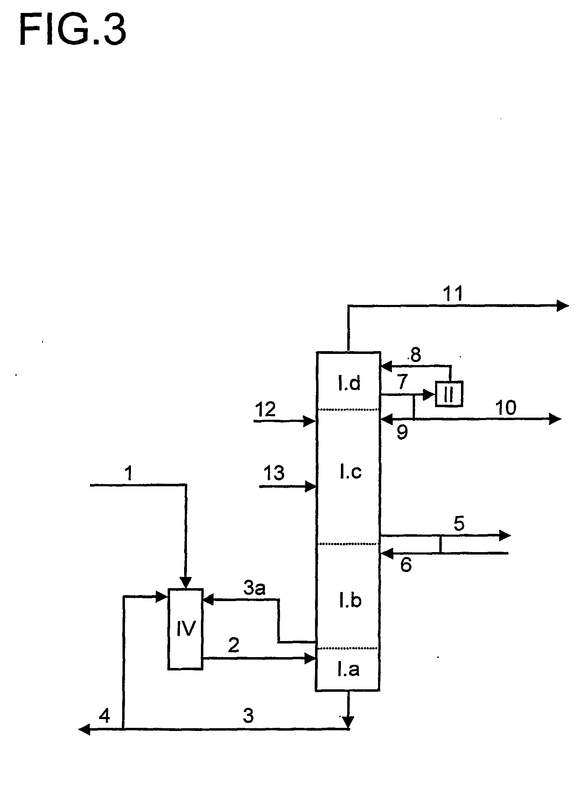

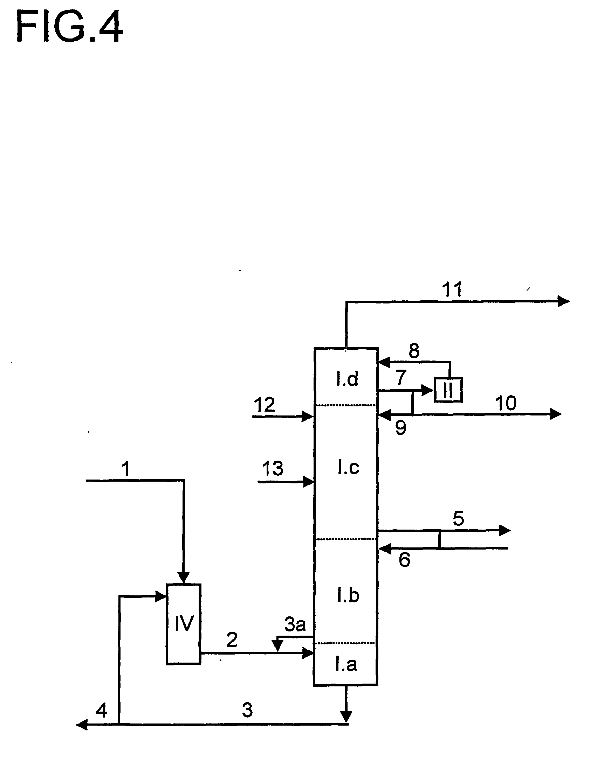

In Abhängigkeit von der Trennaufgabe besteht auch die Möglichkeit auf den Wärmetauscher III zu verzichten (Fig. 3 und Fig. 4).

Das abgekühlte Produktgasgemisch, das in den untersten Bereich Ia einer mit Einbauten versehenen Destillationskolonne eingeleitet wird, wird durch fraktionierende Kondensation in eine oder mehrere Leicht-, Mittel- und Schwersiederfraktionen aufgetrennt, die über Seitenabzüge an den jeweiligen Kolonnenabschnitten abgeführt werden.

Der in der Kolonne herrschende Betriebsdruck beträgt in der Regel 0,5 bis 5 bar (absolut), häufig 0,5 bis 3 bar (absolut) und vielfach 0,5 bis 2 bar (absolut).

Als Kolonneneinbauten kommen prinzipiell alle gängigen Einbauten in Betracht, insbesondere Böden, Packungen und/oder Füllkörper. Von den Böden sind Glockenböden, Siebböden, Ventilböden, Thormannböden und/oder Dual-Flow-Böden bevorzugt. In typischer Weise beträgt bei einer Bodenkolonne die Gesamtzahl an Trennböden 20 bis 80, bevorzugt 50 bis 80.

Weiterhin besteht die Möglichkeit der Kolonne zusätzliche Kühlkreise hinzuzufügen. Dazu wird der Kolonne mittels eines Fangbodens Flüssigkeit entnommen, diese Flüssigkeit mittels eines geeigneten Wärmetauschers gekühlt und die gekühlte Flüssigkeit oberhalb der Abnahmestelle der Kolonne wieder zugeführt (in den Figuren nicht dargestellt).

Selbstverständlich kann in dem Wärmetauscher/Kühlquench-System III/IV auch ein geeignetes Verdünnungsmittel eingesetzt werden. Geeignete Verdünnungsmittel sind polare, unter den Reaktionsbedingungen inerte Lösemittel mit einem Siedepunkt beziehungsweise Siedebereich bei Normaldruck zwischen 180°C und 320°C. Bevorzugt sind Ethylhexansäure, Diphenylether, Nonylphenol, Dibutylformamid. Das Verdünnungsmittel kann bei destillativer Aufarbeitung der ausgeschleusten Schwersiederfraktion (Strom 4) zurückgewonnen und zurückgeführt werden. Ggf. können der Schwersiederfraktion im Wärmetauscher/Kühlquench-System III/IV handelsübliche Dispergierhilfsmittel zugesetzt werden. Geeignet sind anionische, kationische und nichtionische Dispergierhilfsmittel.

Im untersten Kolonnenbereich (Ia, Bereich unterhalb des Schwersiederabzugs) kommt es im wesentlichen zu keiner Kondensation von Schwersiedern (außer gegebenenfalls an Kältebrücken).

Die ausgeschleuste Quenchflüssigkeit (Strom 4) kann vorteilhaft einer thermischen und/oder katalytischen Spaltung unterworfen werden, wobei die rückspaltbaren Wertprodukte, wie z.B. oligomere Acrylsäuren auf bekannte Weise in Acrylsäure gespalten werden. Die Spaltprodukte werden vorteilhaft wieder dem Quench oder der Kolonne zugeführt.

Bevorzugt wird die ausgeschleuste Quenchflüssigkeit (Strom 4) der thermischen Behandlung unterzogen. Die thermische Behandlung wird meist bei 170 bis 190°C und einem Druck von 300 bis 900 mbar durchgeführt. Die Verweilzeit beträgt typischerweise 1 bis 10 Stunden. Die abgetrennten Spaltprodukte werden vorteilhaft wieder dem Quench oder der Kolonne zugeführt. Die Verweilzeit wird bevorzugt durch über die Viskosität des Spaltrückstandes geregelt, so daß der Spaltrückstand bei der Austragstemperatur pumpbar bleibt. Besonders bevorzugt wird der Spaltrückstand getaktet ausgeschleust und mit 10 bis 30 % eines geeigneten Verdünnungsmittels, wie z.B. Methanol verdünnt. Der Stockpunkt des verdünnten Spaltrückstandes beträgt üblicherweise 20 bis 50°C.

Die im Seitenabzug als Mittelsieder entnommene Rohacrylsäure (Strom 5) enthält neben Acrylsäure in der Regel noch

| | |
|---|---|
| 0,1 bis 2 Gew.-% | Niedere Carbonsäuren, zum Beispiel Esssigsäure |
| 0,5 bis 5 Gew.-% | Wasser |
| 0,05 bis 1 Gew.-% | niedermolekulare Aldehyde |
| 0,01 bis 1 Gew.-% | Maleinsäure und/oder deren Anhydrid |
| 1 bis 500 Gew.ppm | Stabilisator, |

jeweils bezogen auf das Gewicht der Rohacrylsäure.

Die als Mittelsiederfraktion entnommene Rohacrylsäure (Strom 5) kann entweder direkt verestert oder zum Zwecke der Weiterreinigung einer Kristallisation zugeführt werden, wobei in diesem Falle die anfallende Mutterlauge vorteilhaft wieder der Kolonne als Rücklauf zugeführt wird (unterhalb des Abzugs der Mittelsiederfraktion, Strom 6).

Eine solche Kristallisation wird in der Regel ohne Zusatz eines Lösungsmittels, insbesondere ohne Zusatz eines organischen Lösungsmittels durchgeführt. Das zu verwendende Kristallisationsverfahren unterliegt keiner Beschränkung. Die Kristallisation kann kontinuierlich oder diskontinuierlich, einstufig oder mehrstufig bis zu nahezu beliebigen Reinheitsgraden durchgeführt werden. Bei Bedarf kann der kristallisativ zu reinigenden Rohacrylsäure vor einer Kristallisation Wasser zugesetzt werden (bezogen auf die enthaltene Menge an Acrylsäure bis zu 10 Gew.-% oder mehr, vorzugsweise bis zu 5 Gew.-%). Ein solcher Zusatz erleichtert die Abtrennung von in der Rohacrylsäure als Nebenprodukt enthaltener niederer Carbonsäure, wie z.B. Essigsäure, da diese im Beisein von Wasser in geringerem Ausmaß in die Acrylsäurekristalle eingebaut wird. Außerdem mindert ein Beisein von Wasser die Verkrustungsneigung im Kristaller.

In Abhängigkeit von der Trennaufgabe besteht auch die Möglichkeit einen Teil der als Mittelsiederfraktion entnommene Rohacrylsäure als zusätzlichen Rücklauf im Kolonnenabschnitt I.b zu verwenden (Strom 6).

Die Abkühlung der Leichtsiederfraktion der Kolonne kann indirekt, beispielsweise durch Wärmetauscher, die dem Fachmann an sich bekannt sind und keiner Beschränkung unterliegen, oder direkt, beispielsweise durch einen Quench, erfolgen, vorzugsweise durch direkte Kühlung.

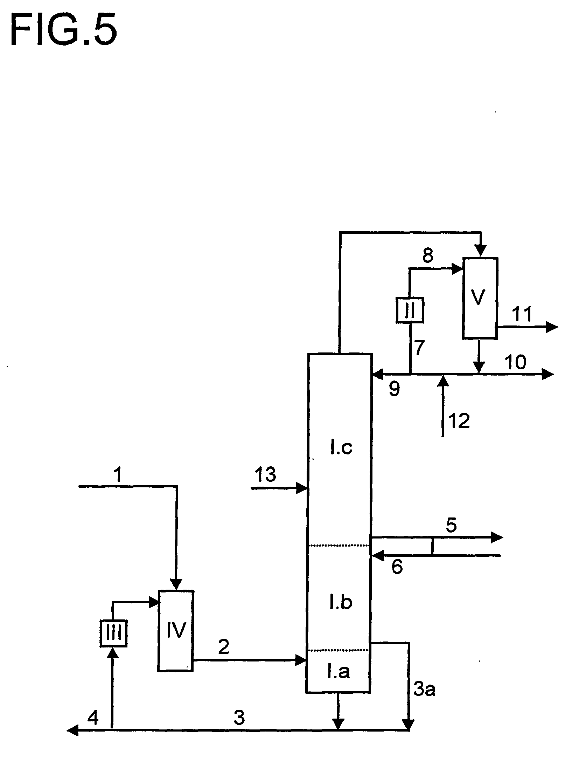

Sie kann sowohl getrennt von der Kolonne in einem separaten Apparat (siehe Figur 5) als auch im Kopfbereich der Kolonne I.d erfolgen, wie es in der Figur 1 gezeigt ist. In diesem Fall wird die kondensierte Leichtsiederfraktion aus dem Kolonnenbereich I.d (Strom 7) in der Regel mit einer Temperatur zwischen 50 und 100 °C dem Kühler II zugeführt.

Zur indirekten Kühlung der kondensierten Leichtsiederfraktion eignen sich alle gängigen Wärmeüberträger oder Wärmetauscher. Als bevorzugt seien Rohrbündelwärmetauscher, Plattenwärmetauscher und Luftkühler genannt. Die Temperatur der kondensierten Leichtsiederfraktion beträgt nach Verlassen des Wärmetauschers II normalerweise 20 bis 60°C, häufig 20 bis 35°C. Geeignete Kühlmedien sind Luft bei einem entsprechenden Luftkühler und Kühlflüssigkeiten, insbesondere Wasser, bei den anderen Kühlvorrichtungen.

Im Rahmen eines Wärmeverbundes kann die Kondensationswärme der Leichtsiederfraktion selbstverständlich auch ganz oder teilweise zum Schmelzen von Acrylsäurekristallen in einer Kristallisation oder zum Verdampfen von Flüssigpropen vor den Reaktoren zur Erzeugung des heißen, Acrylsäure-haltigen Gasgemisches genutzt werden.

Die Leichtsiederfraktion (Strom 7, Sauerwasser) kann teilweise, auf den Kopf der Kolonne zurückgeführt (Strom 8), teilweise als-Rücklauf für den Kolonenabschnitt I.c verwendet (Strom 9) und teilweise ausgeschleust (Strom 10) werden. Sie besteht im Fall der Herstellung von Acrylsäure in der Regel aus

| | |
|---|---|
| 80 - 95 Gew.% | Wasser |
| 2 - 15 Gew.% | Essigsäure |
| 1 - 5 Gew.% | Acrylsäure |
| 0,05 - 1 Gew.% | niederen Aldehyden (wie z.B. Acrolein, Formaldehyd) |

Der Teil der Leichtsiederfraktion, der auf den Kopf der Kolonne zurückgeführt wird, wird üblicherweise so geregelt, daß eine gleichmäßige Gas- und Flüssigkeitsbelastung der trennwirksamen Einbauten der Kolonne und eine optimale Stofftrennung gewährleistet wird. Bevorzugt wird eine sogenannte "modale" Temperaturregelung verwendet: Dazu werden im Bereich eines ausgeprägten Temperatur- bzw. Konzentrationssprunges drei Temperaturmeßstellen installiert (mit den zugehörigen Temperaturen T1, T2 und T3). Ein aus den drei Temperaturmeßstelle erzeugter Wert, z.B. (T1-2×T2+T3), kann dann als Regelgröße für den Kolonnenrücklauf verwendet werden. Weiterhin ist statt der "modalen" Temperaturregelung eine Leitfähigkeitsregelung möglich.

Vorteilhaft kann die Kondensation der Leichtsieder auch in einem außenliegenden Quench V erfolgen (Fig. 5), der mit Sauerwasser betrieben wird. Prizipiell kann dieses Quenchsystem so gestaltet werden, wie für das Quenchsystem IV beschrieben.

Die nicht kondensierbaren Bestandteile des Produktgasgemisches (Stickstoff, Sauerstoff, Propan, Propen, beziehungsweise Isobutan, Isobuten, Kohlenmonoxid, Kohlendioxid etc.) werden am Kopf der Kolonne (Strom 11) ausgeschleust oder bevorzugt, ggf. nach einer Reinigung, zumindest zum Teil als Kreisgas in die Gasphasenoxidation rückgeführt. Besonders bevorzugt ist die Überhitzung des Stromes 11 um 4 bis 10 °C gegenüber der Kopftemperatur der Destillationskolonne, wodurch eine mögliche Kondensation in den Abgas- bzw. Kreisgasleitungen vermieden wird.

Die Temperatur im Sumpf der Kolonne liegt in typischer Weise bei 90 bis 130°C, wohingegen die Kopftemperatur normalerweise 50 bis 100, häufig 60 bis 70°C beträgt.

Die Entnahmetemperatur der Rohacrylsäure (Strom 5) liegt meist bei 80 bis 110°C. Die Rohacrylsäure wird auf 14 bis 20°C vorgekühlt und, falls gewünscht, eniem Kristaller zugeführt. Die Mutterlauge aus einer solchen Kristallisation wird auf 85 bis 95 °C vorgewärmt und in die Kolonne zurückgeführt (Strom 6). Selbstverständlich kann dabei die Wärme des Stromes 5 genutzt werden um den Strom 6 vorzuwärmen (Energieverbund). Besonders bevorzugt wird dabei der bereits vorgekühlte Strom 5 zum Schmelzen von im Kristaller erzeugten Acrylsäurekristallisat verwendet. Die hierfür einsetzbare Wärmetauscher unterliegen keinen Beschränkungen.

Die Rückführtemperatur des Sauerwassers (Strom 8) in die Kolonne liegt in der Regel bei 20 bis 35°C.

Die für die fraktionierende Kondensation einsetzbaren Kolonnen unterliegen keinen besonderen Beschränkungen. Grundsätzlich eignen sich alle Kolonnen mit trennwirksamen Einbauten.

Die Kolonne umfaßt wenigstens eine Kühlvorrichtung. Hierfür eignen sich alle Wärmeübertrager oder Wärmetauscher, bei denen die bei der Kondensation freiwerdende Wärme indirekt (extern) abgeführt wird. Hierfür können alle gängigen Apparate eingesetzt werden, wobei Rohrbündelwärmetauscher, Plattenwärmetauscher und Luftkühler bevorzugt sind. Geeignete Kühlmedien sind bei einem Luftkühler entsprechend Luft und bei anderen Kühlvorrichtungen Kühlflüssigkeiten, insbesondere Wasser. Ist nur eine Kühlvorrichtung vorgesehen, so wird diese bevorzugt am Kopf der Kolonne eingebaut, in dem die Leichtsiederfraktion auskondensiert wird. Der Fachmann kann in Abhängigkeit von der gewünschten Reinheit der kondensierten Fraktionen und damit der Komponenten die Anzahl der erforderlichen Kühlvorrichtungen leicht bestimmen, wobei die Reinheit der kondensierten Komponenten im wesentlichen durch die installierte Trennleistung der Kolonne, d.h. die Kolonnenhöhe, Durchmesser und die über das zu kondensierende Gasgemisch eingetragene Energie bestimmt wird. Zweckmäßigerweise werden bei Vorhandensein mehrerer Kühlvorrichtungen diese in verschiedenen Abschnitten der Kolonne eingebaut. Z.B. können bei einem heißen Gasgemisch, das neben dem hohen Anteil nicht kondensierbarer Komponenten jeweils mindestens eine Schwersieder-, Mittelsieder- und Leichtsiederfraktion enthält, eine Kühlvorrichtung im unteren Abschnitt der Kolonne zur Auskondensierung der Schwersiederfraktion und eine Kühlvorrichtung am Kopf der Kolonne zur Auskondensierung der Leichtsiederfraktion vorgesehen sein. Die kondensierten Fraktionen werden an den jeweiligen Abschnitten in der Kolonne bevorzugt über Seitenabzüge oder Fangböden abgeführt. In Abhängigkeit von der Anzahl der Komponenten in der Schwersieder-, Mittelsieder- und Leichtsiederfraktion können jeweils auch mehrere Seitenabzüge vorgesehen sein. Die über die Seitenabzüge abgezogenen Fraktionen können dann weiteren Reinigungsstufen unterzogen werden, z.B. destillativen oder extraktiven Trennverfahren oder Kristallisation, je nach Natur der Nebenkomponenten und gewünschter Reinheit der Komponenten. In einer bevorzugten Ausgestaltung der Erfindung sind ein Schwersiederabzug, ein Leichtsiederabzug und 1 oder 2 Mittelsiederabzüge vorgesehen.

Der in der Kolonne vorliegende Druck hängt von der Menge an nicht kondensierbaren Komponenten ab und beträgt in der Regel 0,5 - 5 bar Absolutdruck, häufig 0,5 - 3 bar Absolutdruck und vielfach 0,5 - 2 bar Absolutdruck. Die genauen Betriebsbedingungen für die Kolonne, wie Temperatur- und Druckführung, Schaltung und Anordnung der Kühlvorrichtung beziehungsweise Kühlvorrichtungen, Anordnung der Seitenabzüge zum Abziehen der gewünschten Fraktionen, Wahl der Kolonnenhöhe und des Kolonnendurchmessers, Anzahl und Abstand der trennwirksamen Einbauten/Böden in der Kolonne oder Art der trennwirksamen Kolonneneinbauten, können vom Fachmann im Rahmen fachüblicher Versuche in Abhängigkeit von der Trennaufgabe ermittelt werden.

Der Seitenabzug für die Schwersiederfraktion ist am untersten Fangboden, dessen Ausgestaltung nicht beschränkt ist, der Kolonne angebracht. Falls gewünscht können auch mehrere Fangböden für mehrere Schwersiederabzüge verwendet werden, die, mit Ausnahme des untersten Fangbodens, geeignete Überlaufvorrichtungen aufweisen können.

Zweckmäßig ist die Durchführung des erfindungsgemäßen Verfahrens in Gegenwart von an sich bekannten Stabilisatoren von Acrylsäure.

Stabilisatoren im Sinne dieser Schrift sind solche Verbindungen, die die Polymerisation von Acrylsäure verzögern und/oder hemmen.

Als Stabilisatoren sind beispielsweise geeignet phenolische Verbindungen, Amine, Nitroverbindungen, phosphor- oder schwefelhaltige Verbindungen, Hydroxylamine, N-Oxyle und bestimmte anorganische Salze, sowie ggf. Gemische davon in An- oder Abwesenheit von molekularem Sauerstoff.

Bevorzugt sind solche Stabilisatoren wie Phenothiazin, N-Oxyle oder phenolische Verbindungen.

N-Oxyle (Nitroxyl- oder N-Oxyl-Radikale, Verbindungen, die wenigstens eine >N-O• -Gruppe aufweisen), sind z.B. 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-tetramethyl-piperidin-N-oxyl oder 3-Oxo-2,2,5,5-tetramethyl-pyrrolidin-N-oxyl.

Phenolische Verbindungen sind z.B. Alkylphenole, beispielsweise o-, m- oder p-Kresol (Methylphenol), 2-tert.-Butyl-4-methylphenol, 6-tert.-Butyl-2,4-dimethyl-phenol, 2,6-Di-tert.-Butyl-4-methylphenol, 2-tert.-Butylphenol, 4-tert.-Butylphenol, 2,4-di-tert.-Butylphenol, 2-Methyl-4-tert.-Butylphenol, 4-tert.-Butyl-2,6-dimethylphenol, oder 2,2'-Methylen-bis-(6-tert.-butyl-4-methylphenol), 4,4'-Oxydiphenyl, 3,4-Methylendioxydiphenol (Sesamol), 3,4-Dimethylphenol, Hydrochinon, Brenzcatechin (1,2-Dihydroxybenzol), 2-(1'-Methylcyclohex-1'-yl)-4,6-dimethylphenol, 2- oder 4-(1'-Phenyl-eth-1'-yl)-phenol, 2-tert-Butyl-6-methylphenol, 2,4,6-Tris-tert-Butylphenol, 2,6-Di-tert.-butylphenol, 2,4-Di-tert.-butylphenol, 4-tert.-Butylphenol, Nonylphenol [11066-49-2], Octylphenol [140-66-9], 2,6-Dimethylphenol, Bisphenol A, Bisphenol F, Bisphenol B, Bisphenol C, Bisphenol S, 3,3',5,5'-Tetrabromobisphenol A, 2,6-Di-tert-Butyl-p-kresol, Koresin® der BASF AG, 3,5-Di-tert-Butyl-4-hydroxybenzoesäuremethylester, 4-tert-Butylbrenzcatechin, 2-Hydroxybenzylalkohol, 2-Methoxy-4-methylphenol, 2,3,6-Trimethylphenol, 2,4,5-Trimethylphenol, 2,4,6-Trimethylphenol, 2-Isopropylphenol, 4-Isopropylphenol, 6-Isopropyl-m-Kresol, n-Octadecyl-β-(3,5-di-tert-butyl-4-hydroxyphenyl)propionat, 1,1,3-Tris-(2-methyl-4-hydroxy-5-tert-butylphenyl)butan, 1,3,5-Trimethyl-2,4,6-tris-(3,5-di-tert-butyl-4-hydroxybenzyl)benzol, 1,3,5,-Tris-(3,5-di-tert-butyl-4-hydroxybenzyl)isocyanurat, 1,3,5,-Tris-(3,5-di-tert-butyl-4-hydroxyphenyl)-propionyloxyethyl-isocyanurat, 1,3,5-Tris-(2,6-dimethyl-3-hydroxy-4-tert-butylbenzyl)-isocyanurat oder Pentaerythrit-tetrakis-[β-(3,5,-di-tert-butyl-4-hydroxyphenyl)-propionat], 2,6-Di-*tert*.-butyl-4-dimethylaminomethyl-phenol, 6-*sek*.-Butyl-2,4-dinitrophenol, Irganox® 565, 1141, 1192, 1222 und 1425 der Firma Ciba Spezialitätenchemie, 3-(3',5'-Di-*tert*.-butyl-4'-hydroxyphenyl)propionsäureoctadecylester, 3-(3',5'-Di-*tert*.-butyl-4'-hydroxyphenyl)propionsäurehexadecylester, 3-(3',5'-Di-*tert*.-butyl-4'-hydroxyphenyl)propionsäureoctylester, 3-Thia-1,5-pentandiol-bis-[(3',5'-di-*tert*.-butyl-4'-hydroxyphenyl)propionat], 4,8-Dioxa-1,11-undecandiol-bis-[(3',5'-di-*tert*.-butyl-4'-hydroxyphenyl)propionat], 4,8-Dioxa-1,11-undecandiol-bis-[(3'-*tert*.-butyl-4'-hydroxy-5'-methylphenyl)propionat], 1,9-Nonandiol-bis-[(3',5'-di-*tert*.-butyl-4'-hydroxyphenyl)propionat], 1,7-Heptandiamin-bis[3-(3',5'-di-*tert*.-butyl-4'-hydroxyphenyl)propionsäureamid], 1,1-Methandiamin-bis[3-(3',5'-di-*tert*.-butyl-4'-hydroxyphenyl)propionsäureamid], 3-(3',5'-di-*tert*.-Butyl-4'-hydroxyphenyl)propionsäurehydrazid, 3-(3',5'-di-Methyl-4'-hydroxyphenyl)propionsäurehydrazid, Bis(3-*tert*.-Butyl-5-ethyl-2-hydroxy-phen-1-yl)methan, Bis(3,5-di-*tert*.-Butyl-4-hydroxy-phen-1-yl)methan, Bis[3-(1'-methylcyclohex-1'-yl)-5-methyl-2-hydroxy-phen-1-yl]methan, Bis(3-*tert*.-Butyl-2-hydroxy-5-methyl-phen-1-yl)methan, 1,1-Bis(5-tert.-Butyl-4-hydroxy-2-methyl-phen-1-yl)ethan, Bis(5-*tert*.-Butyl-4-hydroxy-2-methyl-phen-1-yl)sulfid, Bis(3-*tert*.-Butyl-2-hydroxy-5-methyl-phen-1-yl)sulfid, 1,1-Bis(3,4-Dimethyl-2-hydroxy-phen-1-yl)-2-methylpropan, 1,1-Bis(5-*tert*.-Butyl-3-methyl-2-hydroxy-phen-1-yl)-butan, 1,3,5-Tris[1'-(3",5"-di-*tert*.-Butyl-4"-hydroxyphen-1"-yl)-meth-1'-yl]-2,4,6-trimethylbenzol, 1,1,4-Tris(5'-*tert*.-Butyl-4'-hydroxy-2'-methyl-phen-1'-yl)butan, Aminophenole, wie z.B. para-Aminophenol, Nitrosophenole, wie z.B. para-Nitrosophenol, p-Nitroso-o-Kresol, Alkoxyphenole, beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol, 3,5-Di-tert-butyl-4-hydroxyanisol, 3-Hydroxy-4-methoxybenzylalkohol, 2,5-Dimethoxy-4-hydroxybenzylalkohol (Syringaalkohol), 4-Hydroxy-3-methoxybenzaldehyd (Vanillin), 4-Hydroxy-3-ethoxybenzaldehyd (Ethylvanillin), 3-Hydroxy-4-methoxybenzaldehyd (Isovanillin), 1-(4-Hydroxy-3-methoxy-phenyl)ethanon (Acetovanillon), Eugenol, Dihydroeugenol, Isoeugenol, Tocopherole, wie z.B. α-, β-, γ-, δ- und ε-Tocopherol, Tocol, α-Tocopherolhydrochinon, sowie 2,3-Dihydro-2,2-dimethyl-7-hydroxybenzofuran (2,2-Dimethyl-7-hydroxycumaran), Chinone und Hydrochinone wie z.B. Hydrochinon, 2,5-Di-tert.-Butylhydrochinon, 2-Methyl-p-hydrochinon, 2,3-Dimethylhydrochinon, Trimethylhydrochinon, 4-Methylbrenzcatechin, tert-Butylhydrochinon, 3-Methylbrenzcatechin, Benzochinon, 2-Methyl-p-hydrochinon, 2,3-Dimethylhydrochinon, Trimethylhydrochinon, 3-Methylbrenzcatechin, 4-Methylbrenzcatechin, tert-Butylhydrochinon, 4-Ethoxyphenol, 4-Butoxyphenol, Hydrochinonmonobenzylether, p-Phenoxyphenol, 2-Methylhydrochinon, 2,5-Di-tert.-Butylhydrochinon, Tetramethyl-p-benzochinon, Diethyl-1,4-cyclohexandion-2,5-dicarboxylat, Phenyl-p-benzochinon, 2,5-Dimethyl-3-benzyl-p-benzochinon, 2-Isopropyl-5-methyl-p-benzochinon (Thymochinon), 2,6-Diisopropyl-p-benzochinon, 2,5-Dimethyl-3-hydroxy-p-benzochinon, 2,5-Dihydroxy-p-benzochinon, Embelin, Tetrahydroxy-p-benzochinon, 2,5-Dimethoxy-1,4-benzochinon, 2-Amino-5-methyl-p-benzochinon, 2,5-Bisphenylamino-1,4-benzochinon, 5,8-Dihydroxy-1,4-naphthochinon, 2-Anilino-1,4,naphthochinon, Anthrachinon, N,N-Dimethylindoanilin, N,N-Diphenyl-p-benzochinondiimin, 1,4-Benzochinondioxim, Coerulignon, 3,3'-Di-tert-butyl-5,5'-dimethyldiphenochinon, p-Rosolsäure (Aurin), 2,6-Di-tert-butyl-4-benzyliden-benzochinon oder 2,5-Di-tert.-Amylhydrochinon.

Aromatische Amine sind z.B. N,N-Diphenylamin, Phenylendiamine sind z.B. N,N'-Dialkyl-para-phenylendiamin, wobei die Alkylreste jeweils unabhängig voneinander 1 bis 4 Kohlenstoffatome enthalten und geradkettig oder verzweigt sein können, Hydroxylamine sind z.B. N,N-Diethylhydroxylamin, phosphorhaltige Verbindungen sind z.B. Triphenylphosphin, Triphenylphosphit oder Triethylphosphit, schwefelhaltige Verbindungen sind z.B. Diphenylsulfid und anorganische Salze sind z.B. Kupfer-, Mangan-, Cer-, Nickel-, Chrom--chlorid, -dithiocarbamat, -sulfat, -salicylat oder -acetat.

Bevorzugt sind Phenothiazin, p-Aminophenol, p-Nitrosophenol, 2-*tert*.-Butylphenol, 4-*tert*.-Butylphenol, 2;4-di-*tert*.-Butylphenol, 2-Methyl-4-*tert*.-Butylphenol, 4-*tert*.-Butyl-2,6-dimethylphenol, Hydrochinon oder Hydrochinonmonomethylether sowie Mangan(II)acetat, Cer(III)carbonat oder Cer(III)acetat, besonders bevorzugt sind Phenothiazin, p-Aminophenol, p-Nitrosophenol, 2-*tert*.-Butylphenol, 4-*tert*.-Butylphenol, 2,4-di-*tert*.-Butylphe-2-Methyl-4-*tert*.-Butylphenol, 4-*tert*.-Butyl-2,6-dimethylphenol, Hydrochinon oder Hydrochinonmonomethylether.

Ganz besonders bevorzugt sind Hydrochinonmonomethylether und ein Gemisch aus Hydrochinonmonomethylether und Phenothiazin.

Die Art der Zugabe des Stabilisators ist nicht beschränkt. Der Stabilisator kann beispielsweise in geeigneter Formulierung an beliebiger Stelle der Kolonne, einem externen Kühlkreislauf oder einem geeigneten Rücklaufstrom zugegeben werden. Bevorzugt ist die Zugabe direkt in die Kolonne oder in einen externen Kühlkreislauf.

Wird ein Gemisch mehrerer Stabilisatoren eingesetzt, so können diese auch unabhängig voneinander in unterschiedlichen Lösungsmitteln gelöst werden.

Die Konzentration des Stabilisators in der Kolonne kann je Einzelsubstanz zwischen 1 und 10000 ppm betragen, bevorzugt zwischen 10 und 5000 ppm, besonders bevorzugt zwischen 30 und 2500 ppm und insbesondere zwischen 50 und 1500 ppm.

Die Stabilisierung der Kolonne erfolgt bevorzugt auf folgender weise:
Der obere Kolonnenbereich wird mit mindestens einer phenolischen Verbindung, vorzugsweise Hydrochinonmonomethylether, stabilisiert.
   Die Zugabe des Stabilisators erfolgt erfindungsgemäß als Schmelze in den oberen Teil des Kolonnenabschnitts I.c (Fig. 1, Strom 12), bevorzugt auf den obersten Boden, und/oder in den Quenchkreislauf I.d/II (nicht gezeigt), wobei eine Konzentration von 50 - 2500 ppm, vorzugsweise 200 - 1500 ppm bezogen auf den Strom, in den eindosiert wird, eingestellt wird.

Bevorzugt handelt es sich dabei um eine Stabilisatormischung, in der die Schmelze mindestens eines Stabilisators mit einem niedrigen Schmelzpunkt von beispielsweise unter 120°C, bevorzugt unter 100 °C, besonders bevorzugt unter 80 °C und insbesondere unter 60°C als Lösungsmittel für mindestens einen Stabilisator mit einem hohen Schmelzpunkt von beispielsweise über 120 °C, bevorzugt über 140 °C, besonders bevorzugt über 160 °C und insbesondere über 180 °C dient.

Besonders bevorzugt wird in der Schmelze mindestens ein weiterer Stabilisator, besonders bevorzugt Phenothiazin, gelöst, wobei eine Konzentration von 1 - 20 Gew%, vorzugsweise 5 - 10 Gew% bezogen auf die Schmelze eingestellt wird.

Selbstverständlich können zusätzlich wasserlösliche Stabilisatoren getrennt als wäßrige Lösungen in den oberen Teil des Kolonnenabschnitts I.c bzw. in den Quenchkreislauf I.d/II dosiert werden.

Der übrige Kolonnenbereich kann mit einer 0,1 - 1,5 Gew.%igen Lösung von Phenothiazin in Acrylsäure stabilisiert werden, wobei die Zugabe bevorzugt in dem Kolonnenbereich erfolgt, in dem die Acrylsäurekonzentration 5 - 20 %, bevorzugt 12 - 18 %, bzw. die Wasserkonzentration 40 - 95 % beträgt, bevorzugt 40 bis 60 % (Strom 13). Die Zugabemenge wird so bemessen, daß der Phenothiazingehalt in der Acrylsäurefraktion (Mittelsiederfraktion, Strom 5) 10 - 1000 ppm, vorzugsweise 50 - 500 ppm beträgt.

Der genannte "obere Kolonnenbereich" ist der Bereich oberhalb des Kolonnenbereichs, in dem Acrylsäure und Wasser in den genannten Konzentrationen vorliegt.

Die Stabilisatorlösungen bzw. Stabilisatorschmelzen können mit Pumpen dosiert werden. Bevorzugt für die Stabilisatorschmelze sind Druckvorlagen. So kann z.B. eine Hydrochinonmonomethylether/Phenothiazin-Schmelze direkt aus dem Aufschmelz- bzw. Vorlagebehälter über ein Regelventil in die Kolonne gedrückt werden. Dazu wird er Behälter mit einem geeigneten Gas, beispielsweise ein stickstoffhaltiges Gas, wie Stickstoff, Luft oder Luft-Stickstoff-Gemische, überdeckt und auf den gewünschten Druck aufgepreßt. Bevorzugt hierfür sind Drücke von 2 bis 10 bar. Besonders bevorzugt wird ein Pufferbehälter verwendet, der die Stabilisatorverorgung übernimmt, wenn der Aufschmelz- bzw. Vorlagebehälter gefüllt wid. Die jeweiligen Behälter, Ventile, Pumpen oder Leitungen können begleitbeheizt werden, um ein Erstarren der Schmelze zu verhindern.

Der Quench IV, in dem das heiße Produktgasgemisch der Oxidationsreaktion auf 100 - 180 °C abgekühlt wird, benötigt in der Regel keine zusätzliche Stabilisierung.

Wenig mit Flüssigkeit benetzte Flächen in der Kolonne werden in der Regel mit stabilisierter Flüssigkeit bedüst. Bevorzugt wird ein Teil der auf den Fangböden entnommenen Flüssigkeit (z.B. Strom 5) oberhalb der Fangböden zu deren zusätzlichen Benetzung eingedüst. Besonders bevorzugt wird ein Teil des Rücklaufes unterhalb der Fangböden zur Benetzung derselben gegen deren Unterseiten gedüst

Es muß als überraschend bezeichnet werden, daß phenolische Verbindungen, besonders Hydrochinonmonomethylether ein gutes Lösungsmittel für Phenothiazin darstellen. Bis zu einer Schmelztemperatur von 100°C können bis zu etwa 20 % Phenothiazin gelöst werden. Trotz der geringen Löslichkeit von Phenothiazin im Sauerwasser führt die erfindungsgemäße Stabilisierung nicht zu Feststoffablagerungen oder Verstopfungen durch Phenothiazin im oberen Kolonnenbereich.

Ein weiterer Gegenstand der Erfindung sind Schmelzen, bestehend aus
a) mindestens einer phenolischen Verbindung,
b) Phenothiazin und
c) gegebenenfalls mindestens einer weiteren Verbindung, die als Stabilisator wirksam ist.

Typische erfindungsgemäße Schmelzen sind wie folgt zusammengesetzt:
a) 60 - 99 Gew%, bevorzugt 80 - 95 % und besonders bevorzugt 90 - 95%,
b) 1 - 20 Gew%, bevorzugt 5 - 15 % und besonders bevorzugt 5 - 10 % und
c) 0 - 20 Gew%, bevorzugt 0 - 15 %, besonders bevorzugt 0 - 5 % und ganz besonders bevorzugt 0%,
wobei die Summe immer 100 Gew% ergibt.

Bevorzugt sind solche Schmelzen, bei denen c) ausgewählt ist unter den oben aufgeführten N-Oxyl-Verbindungen und anorganischen Salzen, besonders bevorzugt unter den N-Oxyl-Verbindungen.

Besonders bevorzugt sind solche Schmelzen, bei denen die phenolische Verbindungen a) ausgewählt sind unter den oben angeführten phenolische Verbindungen, ganz besonders bevorzugt unter p-Aminophenol, p-Nitrosophenol, 2-*tert*.-Butylphenol, 4-*tert*.-Butylphenol, 2,4-di-*tert*.-Butylphenol, 2-Methyl-4-*tert*.-Butylphenol, 4-*tert*.-Butyl-2,6-dimethylphenol, Hydrochinon und Hydrochinonmonomethylether. Insbesondere ist Verbindung a) Hydrochinonmonomethylether.

Der Schmelzpunkt der erfindungsgemäßen Schmelzen liegt in der Regel bei 100 °C oder weniger, bevorzugt sind solche mit einem Schmelzpunkt von 80 °C oder weniger und ganz besonders bevorzugt solche mit einem Schmelzpunkt von 60 °C oder weniger.

Weiterhin ist Gegenstand der Erfindung die Verwendung der erfindungsgemäßen Schmelzen zur Stabilisierung von ethylenisch ungesättigten Verbindungen in Verfahren zu deren Herstellung, wie z.B. Styrol, Acrylnitril, Vinylacetat, Vinylchlorid, Butadien, Isopren oder Chloropren, bevorzugt von α,β-ethylenisch ungesättigten Carbonylverbindungen wie Acrylsäure, Methacrylsäure, Acrolein, Methacrolein, Crotonsäure, Maleinsäure, Maleinsäureanhydrid, Methacrylsäuremethylester, Acrylsäuremethylester, Acrylsäureethylester, Butylacrylat oder 2-Ethylhexylacrylat, besonders bevorzugt von Acrylsäure, Methacrylsäure, Acrolein oder Maleinsäureanhydrid, ganz besonders bevorzugt von Acrylsäure und Methacrylsäure und insbesondere von Acrylsäure.

Das erfindungsgemäße Verfahren ermöglicht eine verbesserte Wirtschaftlichkeit der Aufarbeitung von Acrylsäure durch Verringerung des Aufwandes bei der Bereitung der Stabilisatoransätze sowie einer besseren Stabilisierung im oberen Kolonnenbereich, was zu einer höheren Ausbeute und einer längeren Laufzeit der Kolonne und infolgedessen geringeren Abstellzeiten führt.

Ein weiterer Gegenstand der vorliegenden Erfindung ist ein Verfahren zur rektifikativen Trennung von mindestens eine polymerisierbare Verbindung enthaltenden Stoffgemischen in Gegenwart einer mindestens einen phenolischen Stabilisator enthaltenden Stabilisatorzusammensetzung, wobei die Stabilisatorzusammensetzung als Schmelze in die Rektifikationseinheit dosiert wird.

Polymerisierbare Verbindungen sind ethylenisch ungesättigte Verbindungen, bevorzugt solche ethylenisch ungesättigten Verbindungen, die über einen radikalischen Polymerisationsmechanismus polymerisieren können. Als Beispiele genannt seien Ester der (Meth)acrylsäure mit Alkoholen, die 1 bis 20 C-Atome aufweisen, z.B. (Meth)acrylsäuremethylester, (Meth)acrylsäureethylester, (Meth)acrylsäure-n-butylester, (Meth)acrylsäure-2-ethylhexylester, Vinylaromatische Verbindungen, z.B. Styrol, Divinylbenzol, α,β-ungesättigte Nitrile, z.B. Acrylnitril, Methacrylnitril, α,β-ethylenisch ungesättigte Aldehyde, wie z.B. Acrolein, Methacrolein, Vinylester, z.B. Vinylacetat, Vinylpropionat, halogenierte ethylenisch ungesättigte Verbindungen, z.B. Vinylchlorid, Vinylidenchlorid, konjugierte ungesättigte Verbindungen, z.B. Butadien, Isopren, Chloropren, einfach ungesättigte Verbindungen, z.B. Ethylen, Propylen, 1-Buten, 2-Buten, iso-Buten, cyclische einfach ungesättigte Verbindungen, z.B. Cyclopenten, Cyclohexen, Cyclododecen, N-Vinylformamid, Allylessigsäure, Vinylessigsäure, monoethylenisch ungesättigten Carbonsäuren mit 3 bis 8 C-Atomen sowie deren wasserlöslichen Alkalimetall-, Erdalkalimetall- oder Ammoniumsalze wie beispielsweise: Acrylsäure, Methacrylsäure, Dimethylacrylsäure, Ethacrylsäure, Maleinsäure, Citraconsäure, Methylenmalonsäure, Crotonsäure, Fumarsäure, Mesaconsäure und Itaconsäure, Maleinsäure, N-Vinylpyrrolidon, N-Vinyllactame, wie z.B. N-Vinylcaprolactam, N-Vinyl-N-Alkyl-carbonsäureamide oder N-Vinyl-carbonsäureamide, wie z. B. N-Vinylacetamid, N-Vinyl-N-methylformamid und N-Vinyl-N-methylacetamid, Vinylether, z.B. Methylvinylether, Ethylvinylether, *n-*Propylvinylether, *iso*-Propylvinylether, *n*-Butylvinylether, *sek-*Butylvinylether, *iso*-Butylvinylether, *tert*-Butylvinylether, 4-Hydroxybutylvinylether, oder Vinylphosphonsäure sowie Gemische davon.

Bevorzugt sind (Meth)acrylsäureester, vinylaromatische Verbindungen, halogenierte ethylenisch ungesättigte Verbindungen, monoethylenisch ungesättigte Carbonsäuren und Vinylether, besonders bevorzugt sind (Meth)acrylsäureester, vinylaromatische Verbindungen und monoethylenisch ungesättigte Carbonsäuren, ganz besonders bevorzugt sind (Meth)acrylsäureester und monoethylenisch ungesättigten Carbonsäuren und insbesondere monoethylenisch ungesättigte Carbonsäuren.

Polymerisationsfähige Bestandteile enthaltende Stoffgemische sind solche, bei denen mindestens 50 Gew% der bei Raumtemperatur (25 °C) flüssigen Bestandteile aus polymerisationsfähigen Verbindungen bestehen, bevorzugt mindestens 60 Gew%, besonders bevorzugt mindestens 75 Gew%, ganz besonders bevorzugt mindestens 85 Gew% und insbesondere mindestens 90 Gew%.

Rektifikative Trennung kann darin eine fraktionierende Kondensation eines im wesentlichen gasförmigen Zulaufs oder eine Destillation eines im wesentlichen flüssigen Zulaufs bedeuten.
Die rektifikative Trennung kann an einer Trennkolonne mit beispielsweise 1 bis 150 theoretischen Trennböden erfolgen, bevorzugt 2 bis 120, besonders bevorzugt 5 bis 100, ganz besonders bevorzugt 10 bis 80 und insbesondere 20 bis 50 theoretischen Trennböden.

Die Rektifikationseinheiten sind von an sich bekannter Bauart und weisen die üblichen Einbauten auf. Als Kolonneneinbauten kommen prinzipiell alle gängigen Einbauten in Betracht, beispielsweise Böden, Packungen und/oder Schüttungen. Von den Böden sind Glokkenböden, Siebböden, Ventilböden, Thormannböden und/oder Dual-Flow-Böden bevorzugt, von den Schüttungen sind solche mit Ringen, Wendeln, Sattelkörpern, Raschig-, Intos- oder Pall-Ringen, Barrel- oder Intalox-Sätteln, Top-Pak etc. oder Geflechten bevorzugt.

Die Rektifikationseinheiten weisen in der Regel mindestens einen Kondensator üblicher Bauart, beispielsweise direkte oder indirekte Kühler, bevorzugt Röhren- oder Plattenwärmetauscher oder Quenchkühler, auf, Destillationseinheiten zusätzlich mindestens einen Verdampfer üblicher Bauart, beispielsweise Rohrbündel-, Platten-, Dünnschicht- oder Fallfilmverdampfer.

Die Sumpf- und Kopftemperaturen und -drücke richten sich nach der zu trennenden polymerisierbaren Verbindung. In der Regel wird zur Absenkung der Destillationstemperatur bei verringertem Druck gearbeitet, bei leichtflüchtigen Verbindungen kann die Rektifikation jedoch auch bei erhöhtem Druck durchgeführt werden.

Beispielhafte Kopftemperaturen und -drücke sind für folgende polymerisierbare Verbindungen:

| | Kopftemperatur [°C] | Kopfdruck [mbar] |
|---|---|---|
| Styrol | 50 - 150 | 50 - 1013 |
| Vinylacetat Acrylnitril | 30 - 80 | 200 - 1013 |
| Vinylpropionat | 50 - 100 | 200 - 1013 |
| Methylvinylether | -30 - +10 | 500 - 2000 |
| Ethylvinylether | 0 - 40 | 500 - 2000 |
| 4-Hydroxybutylvinylether | 80 - 190 | 50 - 1013 |
| Methacrylsäure | 80 - 160 | 100 - 1013 |
| Methylmethacrylat | 50 - 100 | 100 - 1013 |
| Acrylsäure | 60 - 140 | 50 - 1013 |
| Methylacrylat | 30 - 80 | 50 - 1013 |
| Ethylacrylat | 40 - 100 | 50 - 1013 |
| Butylacrylat | 70 - 150 | 50 - 1013 |
| 2-Ethylhexylacrylat | 80 - 200 | 50 - 1013 |

Die jeweiligen Sumpftemperaturen liegen in Abhängigkeit von der Zahl der theoretischen Trennböden in der Regel 10 bis 50 °C höher als die Kopftemperatur.

Stabilisatorzusammensetzungen sind solche, bei denen mindestens 50 Gew% der Zusammensetzung als Stabilisator gegen eine Polymerisation, bevorzugt eine radikalische Polymerisation, der zu stabilisierenden polymerisierbaren Verbindung wirksam sind, bevorzugt mindestens 65 Gew%, besonders bevorzugt mindestens 75 Gew% ganz besonders bevorzugt mindestens 90 Gew% und insbesondere 100 Gew%.

Der Schmelzpunkt einer solchen Stabilisatorzusammensetzung liegt bevorzugt über 0 °C und besonders bevorzugt über 25 °C. Der Schmelzpunkt einer solchen Stabilisatorzusammensetzung liegt beispielsweise unter 100 °C, bevorzugt unter 80 °C und ganz besonders bevorzugt unter 60 °C.

Die Stabilisatorzusammensetzungen enthalten beispielsweise mindestens eine der oben aufgeführten phenolischen Verbindungen, bevorzugt p-Aminophenol, p-Nitrosophenol, 2-*tert*.-Butylphenol, 4-*tert*.-Butylphenol, 2,4-di-*tert*.-Butylphenol, 2-Methyl-4-*tert*.-Butylphenol, 4-*tert*.-Butyl-2,6-dimethylphenol, Hydrochinon oder Hydrochinonmonomethylether, besonders bevorzugt 2-*tert*.-Butylphenol, 4-*tert*.-Butylphenol, 2,4-di-*tert*.-Butylphenol, 2-Methyl-4-*tert*.-Butylphenol, 4-*tert*.-Butyl-2,6-dimethylphenol, Hydrochinon oder Hydrochinonmonomethylether, ganz besonders bevorzugt enthalten die Stabilisatorzusammensetzungen eine der oben angeführten erfindungsgemäßen Schmelzen und insbesondere handelt es sich bei der Stabilisatorzusammensetzung um eine der oben angeführten erfindungsgemäßen Schmelzen.

Die Dosierung der Stabilisatorzusammensetzung als Schmelze kann an jedem beliebigen Punkt der Rektifikationseinheit einschließlich der mit dieser verbundenen Leitungen und Geräte, beispielsweise Kondensatoren, Kühler, Verdampfer oder Vakuumeinheiten, erfolgen, bevorzugt erfolgt sie in der oberen Hälfte (bezogen auf die Anzahl der trennwirksamen Einbauten) der Kolonne, besonders bevorzugt in das obere Drittel, ganz besonders bevorzugt in das obere Viertel und insbesondere in das obere Zehntel, dort speziell auf den obersten Boden. In einer weiteren bevorzugten Ausführungsform wird die geschmolzene Stabilisatorzusammensetzung in den Rücklaufstrom gegeben und mit diesem in den oberen Teil der Kolonne geführt.

In dieser Schrift verwendete ppm- und Prozentangaben beziehen sich, falls nicht anders angegeben, auf Gewichtsprozente und - ppm.

Das erfindungsgemäße Verfahren wird durch das nachfolgende Beispiel näher erläutert.

### Beispiel (Fig. 5):

Aus einer heterogen katalysierten Gasphasenoxidation wurde ein 270°C aufweisendes Produktgemisch (Strom 1) der nachfolgenden Zusammensetzung erhalten:

| | |
|---|---|
| 11,5 Gew.-% | Acrylsäure |
| 0,28 Gew.-% | Essigsäure |
| 27 Gew.ppm | Propionsäure |
| 0,093 Gew.-% | Maleinsäureanhydrid |
| 0,1 Gew.-% | Acrolein |
| 0,1 Gew.-% | Formaldehyd |
| 31 Gew.ppm | Furfural |
| 25 Gew.ppm | Benzaldehyd |
| 0,29 Gew.-% | Propen |
| 3,8 Gew.-% | Sauerstoff |
| 5,2 Gew.-% | Wasser |
| 2,8 Gew.-% | Kohlenoxide, und als Restmenge N₂ |

Das Produktgemisch (3600 g/h) wurde in einem Sprühkühler (IV) auf eine Temperatur von 121°C abgekühlt. Als Sprühflüssigkeit wurde die über einen Fangboden aus der Trennkolonne entnommene Schwersiederfraktion (Strom 3) verwendet. Die Sprühflüssigkeit wurde über den mit Wärmeträgeröl betriebenen Rohrbündelwärmetauscher (III) im Kreis geführt. 43 g/h Schwersieder wurden dem Kreislauf kontinuierlich entnommen (Strom 4).

Die Schwersieder (Strom 4) wurden gesammelt. Die darin enthaltenden oligomeren Acrylsäuren wurden diskontinuierlich in einem Rührbehälter in Wertprodukte zurückgespalten (nicht gezeigt). Die Spaltung wurde bei 190°C und einem Druck von 500 mbar durchgeführt. Der Spaltrückstand wurde mit 25 Gew.-% Methanol verdünnt und entsorgt. Das Spaltdestillat wurde gesammelt und kontinuierlich in den Quenchkreis (III/IV) zugeführt. 34 g/h Spaltdestillat wurden zurückgeführt. Das Spaltdestillat enthielt 0,5 - 1,0 Gew.-% Hydrochinonmonomethylether und mußte nicht zusätzlich stabilisiert werden.

Das auf eine Temperatur von 121°C abgekühlte Produktgasgemisch wurde unterhalb des Fangbodens (Kolonnenbereich Ia) der Trennkolonne zugeführt.

Die Kolonne war eine Bodenkolonne mit 45 Dual-flow- und 40 Glokkenböden. Der Boden oberhalb von Boden 15 war als weiterer Fangboden gestaltet. Über ihn wurden 1680 g/h einer eine Temperatur von 101°C aufweisenden Rohacrylsäure ausgeschleust, die folgende Zusammensetzung aufwies (Strom 5):

| | |
|---|---|
| Acrylsäure | 97 Gew.-% |
| Essigsäure | 0,6 Gew.-% |
| Propionsäure | 640 Gew.ppm |
| Furfural | 0,4 Gew.-% |
| Maleinsäureanhydrid | 0,14 Gew.-% |
| Benzaldehyd | 550 Gew.ppm |
| Wasser | 1,5 Gew.-% |

Die Rohacrylsäure wurde einem Suspensionkristaller zugeführt.

Zusätzlich wurden 620 g/h Rohacrylsäure als zusätzlicher Rücklauf auf den Boden 15 gegeben.

Am Kopf der Kolonne wurde ein gasförmiges Gemisch entnommen und im Sprühkühler (V) einer Partialkondensation unterworfen. 482 g/h des dabei anfallenden Sauerwassers, das im wesentlichen aus 5,5 Gew% Acrylsäure, 5,2 Gew% Essigsäure und 85 Gew% Wasser besteht, wurden am Kopf der Kolonne mit einer Temperatur von 30°C in selbige zurückgeführt (Strom 9). 114 g/h des Sauerwassers wurden kontinuierlich entnommen (Strom 10).

Eine Lösung von 5 Gew.-% Phenothiazin in Hydrochinomonomethylether wurde als Schmelze (bei einer Temperatur von 60°C) in einer Menge von 0,5 g/h dem Quenchkreislauf zugeführt (Strom 12).

Eine Lösung von 0,5 Gew.-% Phenothiazin in Acrylsäure wurde in einer Menge von 18 g/h auf dem 47ten Boden der Trennkolonne zugeführt (Strom 13).

Der Kristaller war ein Rührbehälter (3 1 Innenvolumen) mit Wendelrührer. Die Kristallisationswärme wurde über den Doppelmantel des Behälters abgeführt. Die Gleichgewichtstemperatur der Lösung betrug 9,7°C. Die bei der Kristallisation erzeugte Suspension (Feststoffgehalt ca. 30 Gew.-%) wurde auf einer Zentrifuge bei 2000 U/min (Zentrifugendurchmesser 300 mm) und einer Schleuderzeit von 1 min diskontinuierlich in Kristalle und Mutterlauge getrennt. Die Kristalle wurden anschließend mit aufgeschmolzenem (zuvor gewaschen) Kristallisat (134 g/h) 1 min lang bei 2000 U/min gewaschen. Die Mutterlauge wurde zusammen mit der Waschflüssigkeit auf den 15ten Boden in die Trennkolonne zurückgeführt (Strom 6).

Die Analyse der gewaschenen Kristalle (537 g/h) ergab folgende Gehalte:

| | |
|---|---|
| Acrylsäure | 99,7 Gew.-% |
| Essigsäure | 0,14 Gew.-% |
| Propionsäure | 230 Gew.ppm |
| Maleinsäureanhydrid | 72 Gew.ppm |
| Furfural | 210 Gew.ppm |
| Benzaldehyd | 28 Gew.ppm |
| Wasser | 0,11 Gew.-% |

Die beschriebene Trennvorrichtung wies auch nach einer Laufzeit von 1200 h keine nennenswerte Polymerisatbildung auf.

### Vergleichsbeispiel

Es wurde verfahren, wie in Beispiel 2 der DE-A 102 35 847 beschrieben. Nach einer Laufzeit von 1200 Stunden wurde abgestellt und es waren deutliche Ablagerungen durch Polymerisatbildung erkennbar.

## Patentansprüche

1. Verfahren zur fraktionierenden Kondensation eines heißen Gasgemischs, das neben Acrylsäure wenigstens eine weitere kondensierbare Komponente enthält, in einer Kolonne in Anwesenheit mindestens eines Stabilisators, **dadurch gekennzeichnet, daß** der mindestens eine Stabilisator zumindest teilweise als Schmelze dosiert wird.

2. Verfahren nach Anspruch 1, **dadurch gekennzeichnet, daß** die Schmelze mindestens eines Stabilisators mit einem Schmelzpunkt unter 120 °C als Lösungsmittel für mindestens einen Stabilisator mit einem Schmelzpunkt über 120 °C verwendet wird.

3. Schmelze, bestehend aus
a) mindestens einer phenolischen Verbindung,
b) Phenothiazin und
c) gegebenenfalls mindestens einer weiteren Verbindung, die als Stabilisator wirksam ist.

4. Schmelzen gemäß Anspruch 3, **dadurch gekennzeichnet, daß** a) ausgewählt ist unter p-Aminophenol, p-Nitrosophenol, 2-tert.-Butylphenol, 4-*tert*.-Butylphenol, 2,4-di-*tert*.-Butylphenol, 2-Methyl-4-*tert*.-Butylphenol, 4-*tert*.-Butyl-2,6-dimethylphenol, Hydrochinon oder Hydrochinonmonomethylether.

5. Schmelzen gemäß Anspruch 3, **dadurch gekennzeichnet, daß** die Zusammensetzung
a) 60 - 99 Gew%,
b) 1 - 20 Gew% und
c) 0 - 20 Gew%,
wobei die Summe immer 100 Gew% ergibt, beträgt.

6. Verfahren nach einem der Ansprüche 1 - 2, **dadurch gekennzeichnet, daß** eine Schmelze gemäß einem der Ansprüche 3 bis 5 im oberen Kolonnenbereich und Phenothiazin im übrigen Kolonnenbereich zugegeben wird.

7. Verfahren nach einem der Ansprüche 1 - 2 oder 6, **dadurch gekennzeichnet, daß** das heiße Gasgemisch in einem von der Kolonne getrennten Apparat abgekühlt wird.

8. Verfahren nach einem der Ansprüche 1 - 2 oder 6 - 7, **dadurch gekennzeichnet, daß** mindestens ein ausgeschleuster Strom einer thermischen und/oder katalytischen Behandlung unterworfen wird.

9. Verfahren nach einem der Ansprüche 1 - 2 oder 6 - 8, **dadurch gekennzeichnet, daß** es in Gegenwart von molekularem Sauerstoff durchgeführt wird.

10. Verwendung von Schmelzen gemäß einem der Ansprüche 3 bis 5 zur Stabilisierung von ethylenisch ungesättigten Verbindungen in Verfahren zu deren Herstellung.

11. Verfahren zur rektifikativen Trennung von mindestens eine polymerisierbare Verbindung enthaltenden Stoffgemischen in Gegenwart einer mindestens einen phenolischen Stabilisator enthaltenden Stabilisatorzusammensetzung, **dadurch gekennzeichnet, daß** die Stabilisatorzusammensetzung als Schmelze in die Rektifikationseinheit dosiert wird.

## Claims

1. A process for the fractional condensation of a hot gas mixture which as well as acrylic acid comprises at least one further condensable component in a column in the presence of at least one stabilizer, which comprises metering in at least a portion of the at least one stabilizer as a melt.

2. The process according to claim 1, wherein the melt of at least one stabilizer having a melting point below 120°C is used as a solvent for at least one stabilizer having a melting point above 120°C.

3. Melt comprising
a) at least one phenolic compound,
b) phenothiazine and
c) optionally at least one further compound which is effective as a stabilizer.

4. Melts according to claim 3, wherein a) is selected from the group consisting of p-aminophenol, p-nitrosophenol, 2-*tert*-butylphenol, 4-*tert-*butylphenol, 2,4-di-*tert*-butylphenol, 2-methyl-4-*tert*-butylphenol, 4-*tert*-butyl-2,6-dimethylphenol, hydroquinone and hydroquinone monomethyl ether.

5. Melts according to claim 3 which have the following composition:
a): 60 - 99% by weight,
b): 1 - 20% by weight and
c): 0 - 20% by weight,
where the sum thereof is always equal to 100% by weight.

6. The process according to any of claims 1 - 2, wherein a melt according to any of claims 3 to 5 is introduced into the upper column region and phenothiazine into the remaining column region.

7. The process according to any of claims 1 - 2 or 6, wherein the hot gas mixture is cooled in an apparatus isolated from the column.

8. The process according to any of claims 1 - 2 or 6 - 7, wherein at least one discharged stream is subjected to a thermal and/or catalytic treatment.

9. The process according to any of claims 1 - 2 or 6 - 8 which is carried out in the presence of molecular oxygen.

10. The use of melts according to any of claims 3 to 5 for stabilizing ethylenically unsaturated compounds in processes for preparing them.

11. A process for rectificatively separating substance mixtures comprising at least one polymerizable compound in the presence of a stabilizer composition comprising at least one phenolic stabilizer, which comprises metering the stabilizer composition into the rectification unit as a melt.

## Revendications

1. Procédé de condensation fractionnée d'un mélange gazeux chaud qui contient, outre de l'acide acrylique, au moins un composant condensable supplémentaire, dans une colonne en présence d'au moins un stabilisateur, **caractérisé en ce que** le ou les stabilisateurs sont introduits au moins en partie sous la forme d'une masse fondue.

2. Procédé selon la revendication 1, **caractérisé en ce que** la masse fondue d'au moins un stabilisateur ayant un point de fusion inférieur à 120 °C est utilisée en tant que solvant pour au moins un stabilisateur ayant un point de fusion supérieur à 120 °C.

3. Masse fondue, constituée de
a) au moins un composé phénolique,
b) de la phénothiazine et
c) éventuellement au moins un composé supplémentaire, qui agit en tant que stabilisateur.

4. Masses fondues selon la revendication 3, **caractérisées en ce que** a) est choisi parmi le p-aminophénol, le p-nitrosophénol, le 2-tert.-butylphénol, le 4-tert.-butylphénol, le 2,4-di-tert.-butylphénol, le 2-méthyl-4-tert.-butylphénol, le 4-tert.-butyl-2,6-diméthylphénol, l'hydroquinone ou l'éther monométhylique d'hydroquinone.

5. Masses fondues selon la revendication 3, **caractérisées en ce que** la composition est
a) 60 à 99 % en poids,
b) 1 à 20 % en poids et
c) 0 à 20 % en poids,
la somme étant toujours de 100 % en poids.

6. Procédé selon l'une quelconque des revendications 1 à 2, **caractérisé en ce qu'**une masse fondue selon l'une quelconque des revendications 3 à 5 est introduite dans la zone supérieure de la colonne et de la phénothiazine dans la zone restante de la colonne.

7. Procédé selon l'une quelconque des revendications 1 à 2 ou 6, **caractérisé en ce que** le mélange gazeux chaud est refroidi dans un appareil séparé de la colonne.

8. Procédé selon l'une quelconque des revendications 1 à 2 ou 6 à 7, **caractérisé en ce qu'**au moins un courant déchargé est soumis à un traitement thermique et/ou catalytique.

9. Procédé selon l'une quelconque des revendications 1 à 2 ou 6 à 8, **caractérisé en ce qu'**il est réalisé en présence d'oxygène moléculaire.

10. Utilisation de masses fondues selon l'une quelconque des revendications 3 à 5 pour la stabilisation de composés éthyléniquement insaturés dans leur procédé de fabrication.

11. Procédé de séparation rectificative de mélanges de substances contenant au moins un composé polymérisable en présence d'une composition de stabilisation contenant au moins un stabilisateur phénolique, **caractérisé en ce que** la composition de stabilisation est introduite sous la forme d'une masse fondue dans l'unité de rectification.
